# EUROPEAN PATENT APPLICATION

(11) **EP 3 646 884 A1**
(43) Date of publication of application: **06.05.2020**
(21) Application number: 18306421.1
(22) Date of filing: 30.10.2018
(51) Int. Cl.: A61K 39/395, A61P 43/00, G01N 33/50

(54) **METHOD FOR ESTIMATING SURVIVAL OF COLCHICINE-INTOXICATED PATIENTS**

(71) Applicant: ASSISTANCE PUBLIQUE, HOPITAUX DE PARIS, 75004 Paris (FR); Université Paris Descartes, 75270 Paris Cedex 06 (FR); Université Paris Diderot - Paris 7, 75205 Paris 13 (FR)
(72) Inventor: BAUD, Frédéric, 95160 MONTMORENCY (FR); VICAUT, Éric, 75019 PARIS (FR); DAMBOISE, Viviane, 88400 XONRUPT-LONGEMER (FR)
(74) Representative: Flesselles, Bruno F.G.

(57) **Abstract**

The invention relates to a new non-invasive test making it possible to estimate the likelihood of death of patients intoxicated with colchicine, depending in particular on the time of intoxication or entry at the hospital.

## Description

The invention relates to a new non-invasive test making it possible to estimate the survival of patients intoxicated with colchicine, by using in particular the time of intoxication or, when not known the entry at the hospital.

Colchicine is a medication to treat gout. In addition to gout, familial Mediterranean fever, pericarditis, and Behçet's disease. It has also demonstrated efficacy for prevention of atrial fibrillation after cardiac surgery.

It has a narrow therapeutic index, resulting in severe toxicity after overdose, especially after intravenous (IV) administration or intentional poisoning.

Poisoning (adverse effects) result primarily in initial gastrointestinal upset at high doses that last for around 24 h and may be followed by multiorgan dysfunction or death from cardiogenic shock, usually within 48-96 h.

Colchicine inhibits the deposition of uric acid crystals and is an inhibitor of mitosis. As indicated above, nausea, vomiting, abdominal pain, and diarrhea, with a massive loss of fluid and electrolytes are the first clinical symptoms of colchicine poisoning. An acute dose of about 0.8 mg/kg of colchicine is presumed to be fatal.

The conventional treatment when facing intoxication with colchicine is stomach lavage and rapid gastric decontamination with activated charcoal.

It is also important to initiate rehydration of the patient in response to the diarrhea and vomiting and to correct the hydroelectric troubles.

One can also administer labile blood products to correct any anemia and/or hemostatic troubles.

In case of a suspected infection or of aplastic anemia (bone marrow aplasia), one can also administer antibiotics.

Catecholamines, including dobutamine, can also be used in case of cardiovascular shock, depending on the mechanisms of shock.

However, despite these lines of treatment (conventional ways of treating colchicine), a certain number of patients eventually die from the cardiogenic shock that follows the massive organ failure. Indeed, after admission to the hospital, and in response to the treatment, the patient's health status may improve, stabilize or worsen.

There is no licensed therapy, with current care revolving around supportive intensive care as described above.

Recently, Fab fragments against colchicine (Anti-colchicine Fab referenced as ColchiBIND) have been developed by Micropharm Ltd (Carmarthenshire, United Kingdom) and showed clinical efficacy (preventing lethal colchicine toxicity) in a porcine model (Eddleston et al, Clin Toxicol (Phila). 2018 Aug;56(8):773-781). This confirms data already obtained in murine models, showing increased clearance in rats (Peake ey al Clin Toxicol (Philadelphia, Pa). 2015;53:427-432). Colchicine-specific Fab fragments have also proven able to treat severe overdose in human (Baud et al, N Engl J Med 1995; 332:642-645), and shall prevent cardiogenic shock.

However, these Fab fragments are not easy to produce in quantity (they are produced in ovine (goat or sheep) animals), are expensive and can't be administered to any and all patients that are admitted in hospital with colchicine intoxication.

It is thus important to be able to monitor the patients in order to predict whether they will respond to the conventional treatment or whether they may die from the intoxication, to be able to treat the patients with the anti-colchicine Fab fragments only if there is a vital risk for the patient, and be able to initiate treatment as soon as the patient is declared non-respondent.

Although the dose ingested has an influence on the clinical picture, it is not, by itself, enough to allow prediction of the death of the patient. Indeed, the mortality rate in the three groups of supposed ingested dose equal to or less than 0.35 mg/kg, >0.35 - equal or less than 0.7, and > 0.7 were 0 (0%), 6/33 (18%), and 12/36 (33%) (Fisher's exact test: p < 0.0001). The relation of mortality rate to the dose is statistically highly significant albeit being weak. Therefore, from a clinical viewpoint, other parameters reflecting the effects of the dose should be considered to define the likelihood of death in colchicine overdose.

The inventors have thus developed a test that is able to predict the risk of death of a patient by a simple dosing of circulating biomarkers and combining the values thereby measured in a function in order to obtain an end result which, if above a given threshold, is indicative of a risk of death for the patient (in the absence of treatment). This test is thus able to detect the patients that don't respond to the conventional treatment, regardless of the dose ingested.

In particular, the biomarkers are white blood cell count (GB), plasma blood urea nitrogen (UREA), platelet count (PI) and blood bicarbonate ions (alkaline reserve, RA).

In a preferred embodiment, the biomarkers are GB, PI and UREA.

In another embodiment, the biomarkers are GB, PI and RA.

It is to be noted that the ingested dose could also be used as a parameter in the test as developed below. It could also replace one of the above parameters, in particular platelet count. However, it is not always possible to know it and the tests herein disclosed thus don't take this parameter into consideration. However, it could be possible to include this parameter in another test that would provide the same kind of information and output than the ones herein disclosed, using the methods for obtaining a test that are disclosed below.

The clinical situation of a patient intoxicated with colchicine continuously and rapidly evolves and the inventors have thus adapted to this situation. Consequently, the inventors have proposed various formulas that are adapted to this disease evolution, and that depend on the time from intoxication or, when such time is not known, the time from admission in the hospital. In an ideal situation, the intoxication moment would be known, but since it is not always the case, some tests were also developed without this information. As can be seen later below, these tests have similar qualities, as shown by the AUROC value.

It is indeed important to make sure to obtain a result that is as accurate as possible in order to be able to initiate treatment with Fab fragments as soon as possible and when needed.

The inventors thus proposed 7 formulas that have to be implemented within the first hours of the patient being at the hospital, each formula being linked to a specific length of time from the intoxication or the admission at the hospital.

The quality of a test is determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be acknowledged as a curve allowing prediction of the quality of a test. It is best for the AUROC to be as closed as 1 as possible, this value describing a test which is 100 % specific and sensitive.

It is reminded that
(1) sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).
(2) specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).
(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).
(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

In the tests disclosed in the present document, it was possible to achieve a 100 % sensitivity (*i.e.* all patients above the threshold would die despite conventional treatment) for 6 out of 7 formulas herein proposed. Specificity was also very high (*i.e.* there was a very small number of patients who would survive without Fab treatment and who had a score above the threshold).

Such high levels of specificity and sensitivity are reflected in the AUROC, six out of seven having a value about 096 (the last one having an AUROC of 0.9).

Generally, a diagnosis method comprises
i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

As a matter of illustration
i. In the present case, the information obtained from the patient is
   - The time of intoxication, or the time of admission
   - The levels (amount) of blood biomarkers
   - once the above information is obtained, the values of the biomarkers amounts are combined in a function that depends on the time as recorded and an end result is obtained
ii. the end result us compared to a threshold (depending on the function that is used).
iii. the last step is actually making the diagnosis/prognosis i.e. deciding whether or not the patient has the condition sought (risk of dying) resp. whether or not the patient will evolve to worsening and eventual death. Although the function proposed herein are sufficient by themselves, the physician may also take into account other elements (such as the consistency of the information gathered or the like) to make the diagnostic/prognosis.

Consequently, in the methods disclosed in the present application, step i. also includes a step i.a), which comprise the steps of modifying the information obtained from the patient in order to obtain a new type of information, which is the end result that is then compared to the standards (thresholds) in step ii. Such modification is the combination of the values of variables in a function, and obtaining an end value.

It is further to be noted that the mere measurement of the values of levels of markers in the plasma or serum of a patient and the combination thereof in an algorithm as herein disclosed is part of a method but only provides an intermediate result (an end value or index) that would then to be compared to a reference index (threshold), in order to actually be able to pose the diagnostic.

It is also to be noted that the tests herein disclosed are not "gold-standard" tests, in the sense that the output (index calculated by the formulas herein disclosed) isn't a definitive answer as to the state of the patient. Indeed, these tests are based on statistics and although the sensitivity and sensibility are very high, there may be false-positive or false-negative results, which is the reason why the specific experience of the physician in interpreting the index is of importance for making the prognosis and deciding which kind of follow up is to be made to ne made for each patient.

However, due to the specificity, sensitivity, positive predictive value and negative predictive value of the tests, these tests are of great interest by themselves in provided a help to the physician when investigating a clinical case. Consequently, step iii as disclosed above is not direct and immediate from step ii, as the physician must interpret the result from the clinical and general context to be able to reach a conclusion.

In the present application
"Time of intoxication" indicates the moment when the patient has taken an overdose of colchicine. When available, this information is generally obtained directly from the patient.

"Time of admission to the hospital" indicates the moment when the patient is admitted in a point of care (generally an emergency service in a hospital). This moment is thus recorded in the hospital books.

"MarkerX_Max_YYh" represents the maximal value measures for marker X during the previous YY hours. As an illustration, if YY = 36 and values of marker X have been measured at 12, 24 and 36 hours, MarkerX_Max_36h is the highest value among the values measured at 12, 24 and 36 hours.

"MarkerX_Min_YYh" represents the minimal value measures for marker X during the previous YY hours. As an illustration, if YY = 36 and values of marker X have been measured at 12, 24 and 36 hours, MarkerX_Min_36h is the lowest value among the values measured at 12, 24 and 36 hours.

GB corresponds to the white blood cells count per mm³.

UREA corresponds to the value of the urea (blood urea nitrogen) as measured in the plasma of the patient (mmol / l)

PI corresponds to the platelet count (per mm³)

RA corresponds to the alkali reserve (blood bicarbonate ions in mmol/l) in the blood of the patient. Alternatively, it is possible to use the total blood CO₂ (mmol/l) instead of blood bicarbonate.

All these parameters are known in the art and various methods are available to measure them.

The invention thus relates to a method for determining whether a patient intoxicated with colchicine will die comprising the steps of
(a) combining the values of at least three markers in a function, in order to obtain an end value,
   ∘ wherein the three markers comprise GB, PI, and at least one of UREA and RA
   ∘ wherein the coefficients of the function depends on the time of intoxication or of hospital admission
(b) comparing the end value to a predetermined value, and
(c) determining that the patient is susceptible to die if the end value is higher that the predetermined value,

The predetermined value in step b) depends on the function that is used. It is to be noted that the method is particularly interesting to determine the risk of death by cardiogenic shock for patients that receive the conventional treatment as described above (rehydration, active charcoal, and potentially labile blood products, antibiotics and/or dobutamine).

This method thus makes it possible to assess or evaluate the risks for the patient to die. In particular, the death shall be due to cardiogenic shock. Consequently, the method allows detecting the risk for the patient to die from the colchicine intoxication.

In a specific embodiment, the patient will be treated by anti-colchicine antibodies (which can be injected or orally administered) if risk of death of the patient is predicted.

It is to be noted that the method herein described has a prognosis value. Indeed, if the end value is higher than the appropriate threshold, one can predict that the patient will die (as indicated above, the sensitivity of the proposed tests is 100% for 6 out of 7). Furthermore, since the inventors propose to use the method regularly (with adapted functions depending on the time), this ensures that appropriate treatment (administration of Fab fragments) can be administered as early as possible.

In particular, the function has been obtained by
i) classification of a cohort of patients in different groups according to the occurrence of death of the patients, and further taking into account the time of intoxication and/or hospital admission
ii) identification and selection of factors (amount of biochemical markers) which differ significantly between these groups by unidimensional analysis, wherein at least white blood cell count (GB), platelet count (PI) and at least one of plasma blood urea nitrogen (UREA) and blood bicarbonate ions (alkaline reserve, RA) are selected in this step
iii) logistic regression or Cox regression analysis assessing the independent discriminative value of markers for occurrence of death of the patient
iv) construction of the function by combination of these identified independent factors to obtain a Cox or logistic function.

Such method is also a subject of the invention. The dose may also be used as a factor in step ii, with the other listed factors or replacing one of these (in particular platelet count).

In the preferred embodiment, the function is a linear function. The coefficients of the function depend on the time of intoxication or of hospital admission. The function has preferably been obtained by a logistic regression (and is called a logistic function).

In particular, the function has been obtained by:
i) classification of a cohort of patients in different groups according to the occurrence of death of the patients, and further taking into account the time of intoxication and/or hospital admission
ii) identification of factors (amount of biochemical markers) which differ significantly between these groups by unidimensional analysis
iii) logistic regression analysis to assess the independent discriminative value of markers for occurrence of death
iv) construction of the logistic function by combination of these identified independent factors.

Combination of the values within the obtained function provides an end result that is predictive of death depending of a threshold that depends on the function obtained.

The most favorable threshold is then determined as the one providing the best (highest) AUROC when plotting the ROC for each possible threshold.

It is also possible to normalize the function as to have end values vary between 0 and 1.

In another embodiment, the function is a (multivariate) Cox regression (the hazard being the occurrence of death of the patient).

Such Cox function is preferably obtained by:
a) Assessing / evaluating the death of a patient (in particular due to cardiogenic shock) with the traditional treatment of colchicine intoxication after a given duration of time (generally during the first 96h after ingestion in patients with or without chronic liver diseae, wherein the values of the variables used in the function (as defined above) are known at the beginning of the duration of time
b) Performing a multivariate Cox regression by combination of said values, on the basis of the occurrence of death after the given duration of time
c) Assessing the independent discriminative value of the values by analysis of the multivariate Cox regression,
d) Combining the relative weight of the values of each marker, as individually determined in the multivariate Cox regression, with a negative sign when the markers harbor a negative correlation with the observation of death occurrence.

The markers are the same as disclosed above (comprise at least three markers selected from GB, PI, and at least one of UREA and RA, and potentially other ones such as the dose of ingested colchicine).

The markers that are used in the various functions and selected for obtaining either the logistic or Cox function include at least white blood cell count (GB), platelet count (PI) and at least one of plasma blood urea nitrogen (UREA) and blood bicarbonate ions (alkaline reserve, RA). Colchicine dose may also be used. The value of a marker that is considered for constructing the function is either the value measured at this specific time or a value that has been measured prior hand. Consequently, and as seen above, the function may contain values of markers that have been measured at different times.

This method is performed *in vitro* or *ex vivo.* It can be computer-assisted or computer-implemented.

When the method is computer-implemented, it may comprise the following step:
a) input data is recorded (inputed by an operator) in an electronic form on an local computer, wherein said input data comprises
   i. time of intoxication and/or time of admission at the hospital
   ii. values of biochemical markers as measured in the patient,
b) said input data is sent to a distant server
c) the end value is calculated on the distant server, according to the methods herein disclosed and
d) the information relating to the risk of death is sent back to the local computer.

Optionally, the end value is also sent.

Alternatively, one can send the end value only together with instructions as to how to interpret such end value.

It is preferred when the communications between said the local computer and the distant server are encrypted.

As indicated above, the inventors designed multiple functions depending on the time of intoxication or of the time of admission at the point of care, to reflect on the rapidly evolving nature if the health status of the patient and thus to make sure that the results indicating the risk of worsening of the health status of the patient are as accurate as possible.

Consequently, the function may be selected from the group consisting of

### a) When the time of intoxication is known

### i. if the time of intoxication is shorter than 24 hours

FI<24 = a1 + a2 GB_Max_24h - a3 PI_Min_24h + a4 UREA_Min_24h
with -3.5<a1<-3.4, 0.0002<a2<0.00026, 0.00002<a3<0.00004, 0.65<a4w0.75.

In particular, FI<24 = -3.4760 + 0.000236 GB_Max_24h -0.00003 PI_Min_24h + 0.6961 UREA_Min_24h

### ii. if the time of intoxication is between 24 and 36 hours

FI<36 = b1 + b2 GB_Min - b3 PI Min + b4 UREA_Max
with -1.10<b1<-1.00, 0.0002<b2<0.00032, 0.00002<b3<000004, 0.27<b4<0.37

In particular, FI<36 = -1.0604 + 0.000272 GB_Min -0.00003 PI Min + 0.3268 UREA_Max

### iii. if the time of intoxication is between 36 and 48 hours

FI<48 = c1 + c2 GB MA - c3 PI Min + c4 UREA_Max
with -4.5<c1<-5.5, 0.00015<c2<0.00025, 0.00001<c3<0.00003, 0.28<c4<0.38.

In particular, FI<48 = -4.4512 + 0.000193 GB MA -0.00002 PI Min + 0.3317 UREA_Max

### b) When the time of intoxication is not known and the time of admission at the hospital is known

### i. if the entry at the hospital is less than 12 hours

FA<12 = d1 + d2 GB_Max_12h - d3 PI_Min_12h - d4 RA_Min_12h
with 2.0<d1<2.5, 0.0002<d2<0.00025, 0<000005<d3<0.000015, 0.15<d4<0.25

In particular, FA<12 = 2.2498 + 0.000222 GB_Max_12h -0.00001 PI_Min_12h -0.2078 RA_Min_12h

### ii. if the entry at the hospital is more than 12 hours and less than 24 hours

FA<24 = e1 + e2 GB_Max_24h - e3 PI_Min_24h + e4 UREA_Min_24h
with -3.5<e1<-3.0, 0.00015<e2<0.00025, 0.00001<e3<0.00003, 0. 31<e4<0.41
in particular, FA<24 = -3.2914 + 0.000197 GB_Max_24h -0.00002 PI_Min_24h + 0.3682 UREA_Min_24h

### iii. if the entry at the hospital is more than 24 hours and less than 36 hours

FA<36 = f1 + f2 GB_Max_36h - f3 PI_Min_36h + f4 UREA_Max_36h
with -5.25<f1<-5.1, 0.00012<f2<0.00022, 0.00001<f3<0.00003, 0.36<f4<0.047
In particular, FA<36 = -5.1716 + 0.000170 GB_Max_36h -0.00002 PI_Min_36h + 0.4248 UREA_Max_36h

### iv. if the entry at the hospital is more than 36 hours and less than 48 hours

FA<48 = g1 + g2 GB_Max_48h - g3 PI_Min_48h + g4 UREA_Max_48h
with -5.97<g1<-5.77, 0.00010<g2<0.00020, 0.000005<g3<0.000015, 0.38<g4<0.48
In particular, FA<48 = -5.8761 + 0.000153 GB_Max_48h -0.00001 PI_Min_48h + 0.4370 UREA_Max_48h

It is thus preferred when the function is selected in the group selected from

### a) When the time of intoxication is known

i. FI<24 = -3.4760 + 0.000236 GB_Max_24h -0.00003 PI_Min_24h + 0.6961 UREA_Min_24h
ii. FI<36 = -1.0604 + 0.000272 GB_Min -0.00003 PI Min + 0.3268 UREA_Max
iii. FI<48 = -4.4512 + 0.000193 GB MA -0.00002 PI Min + 0.3317 UREA_Max

### b) When the time of intoxication is not known and the time of admission at the hospital is known

i. FA<12 = 2.2498 + 0.000222 GB_Max_12h -0.00001 PI_Min_12h - 0.2078 RA_Min_12h
ii. FA<24 = -3.2914 + 0.000197 GB_Max_24h -0.00002 PI_Min_24h + 0.3682 UREA_Min_24h
iii. FA<36 = -5.1716 + 0.000170 GB_Max_36h -0.00002 PI_Min_36h + 0.4248 UREA_Max_36h
iv. FA<48 = -5.8761 + 0.000153 GB_Max_48h -0.00001 PI_Min_48h + 0.4370 UREA_Max_48h

The invention also relates to anti-colchicine antibodies or fragments thereof for their use for the treatment of a patient intoxicated with colchicine, wherein application of the methods herein disclosed indicate that the patient is susceptible to make an adverse effect, namely to die in particular from cardiogenic shock. In particular, such anti-colchicine antibodies are to be used for patients for which the method indicates a risk of death. This is when the end value obtained for the patient by the methods herein disclosed is above the threshold for the considered function.

In one embodiment, the antibodies are polyclonal antibodies.

In another embodiment, the antibodies are monoclonal antibodies. Monoclonal antibodies can easily be obtained by isolating a B cell-secreting an antibody of interest immortalizing such cell by methods known in the art.

In another embodiment, the antibodies that are used for treating the patient are selected from the group consisting of Fab fragments, Fab' fragments, F(ab')2 fragments, Fv fragments, diabodies, single chain antibody molecules and other antibody fragments as long as they exhibit the desired capability of binding to colchicine.

It is preferred when the antibody fragments are Fab fragments (i.e. the regions on an antibody that binds to antigens, composed of one constant and one variable domain of each of the heavy and the light chain).

In particular, these Fab fragments form a polyclonal mixture and have been obtained from a polyclonal composition (for instance by enzymatic cleavage) isolated from ovine animals, such as goat or sheep.

### EXAMPLES

### Example 1

The study was performed on a cohort of 135 patients intoxicated with colchicine, 113 of whom survived and 22 of who died.

Intoxication was with only colchicine only for 31 patients (23.0%) and colchicine and at least one other drug for 104 patients (77.0%). Such proportions were the same in the group of surviving patients and the group of patient who died.

Various biomarkers were analyzed and seven functions were designed according to the duration from intoxication or from admission at the hospital.

These functions are

| **Time of assessment** | **Function** | **Threshold** | **AUROC** |
|---|---|---|---|
| Intoxication<24h | FI<24 = -3.4760 + 0.000236 GB_Max_24h -0.00003 PI_Min_24h + 0.6961 UREA_Min_24h | 0.10781 | 0.9605 |
| Intoxication<36h | FI<36 = -1.0604 + 0.000272 GB_Min - 0.00003 PI Min + 0.3268 UREA_Max | 0.49328 | 0.9549 |
| Intoxication<48h | FI<48 = -4.4512 + 0.000193 GB_Max -0.00002 PI Min + 0.3317 UREA_Max | 0.1128 | 0.9637 |
| Admission<12h | FA<12 = 2.2498 + 0.000222 GB_Max_12h -0.00001 PI_Min_12h - 0.2078 RA_Min_12h | 0.41082 | 0.9067 |
| Admission<24h | FA<24 = -3.2914 + 0.000197 GB_Max_24h -0.00002 PI_Min_24h + 0.3682 UREA_Min_24h | 0.10302 | 0.9644 |
| Admission<36h | FA<36 = -5.1716 + 0.000170 GB_Max_36h -0.00002 PI_Min_36h + 0.4248 UREA_Max_36h | 0.11976 | 0.9693 |
| Admission<48h | FA<48 = -5.8761 + 0.000153 GB_Max_48h -0.00001 PI_Min_48h + 0.4370 UREA_Max_48h | 0.13549 | 0.9681 |

The AUROC given above have been calculated with regards to the specified thresholds. The physician may use other thresholds if it is desire to increase the sensitivity (increase the threshold value) or decrease the specificity (decrease the threshold value).

The above methods and functions thus provide a help to the physician to decide whether to bring anti-colchicine antibodies (in particular anti-colchicine Fab fragments) at the patient's bed and whether to administer such. In view if the rarity and of the cost of such products, the physician shall also take into account any other clinical element, such as the existence of the conventional treatment as described above, the delay since the intoxication, the ingested dose or other clinical features.

The methods make it possible to predict whether the conventional treatment is efficient or not for the patient and whether the patient is about to die despite the conventional treatment. It may not be as efficient for prediction if such treatment has not been initiated in due time.

## Claims

1. An *in vitro* method for determining whether a patient intoxicated with colchicine is to die, comprising the steps of
(a) combining the values of at least three markers in a function, in order to obtain an end value,
∘ wherein the three markers are white blood cell count (GB), platelet count (PI) and at least one of plasma blood urea nitrogen (UREA) or blood bicarbonate ions (alkaline reserve, RA)
∘ wherein the coefficients of the function depends on the time of intoxication or of hospital admission
(b) comparing the end value to a predetermined value [wherein the predetermined value depends on the function), and
(c) determining that the patient is susceptible to die if the end value is higher that the predetermined value.

2. The method of claim 1, wherein the patient has been treated for colchicine intoxication by at least one of stomach lavage, administration of activated charcoal, and rehydration.

3. The method of claim 1 or 2, wherein the function also comprises the amount of ingested colchicine as a marker.

4. The method of any one of claims 1 to 3, wherein the function is a logistic function obtained by logistic regression.

5. The method of any one of claims 1 to 3, wherein the function is a Cox function.

6. The method of any one of claims 1 to 5, wherein the function has been obtained by
i) classification of a cohort of patients in different groups according to the occurrence of death of the patients, and further taking into account the time of intoxication and/or hospital admission
ii) identification and selection of factors (amount of biochemical markers) which differ significantly between these groups by unidimensional analysis, wherein at least white blood cell count (GB), platelet count (PI) and at least one of plasma blood urea nitrogen (UREA) and blood bicarbonate ions (alkaline reserve, RA) are selected in this step
iii) logistic regression or Cox regression analysis assessings the independent discriminative value of markers for occurrence of death of the patient
iv) construction of the function by combination of these identified independent factors to obtain a Cox or logistic function.

7. The method of any one of claims 1 to 4 or 6, wherein the function is selected in the group selected from
a) When the time of intoxication is known
i. FI<24 = -3.4760 + 0.000236 GB_Max_24h -0.00003 PI_Min_24h + 0.6961 UREA_Min_24h
ii. FI<36 = -1.0604 + 0.000272 GB_Min -0.00003 PI Min + 0.3268 UREA_Max
iii. FI<48 = -4.4512 + 0.000193 GB MA -0.00002 PI Min + 0.3317 UREA_Max
b) When the time of intoxication is not known and the time of admission at the hospital is known
i. FA<12 = 2.2498 + 0.000222 GB_Max_12h -0.00001 PI_Min_12h -0.2078 RA_Min_12h
ii. FA<24 = -3.2914 + 0.000197 GB_Max_24h -0.00002 PI_Min_24h + 0.3682 UREA_Min_24h
iii. FA<36 = -5.1716 + 0.000170 GB_Max_36h -0.00002 PI_Min_36h + 0.4248 UREA_Max_36h
iv. FA<48 = -5.8761 + 0.000153 GB_Max_48h -0.00001 PI_Min_48h + 0.4370 UREA_Max_48h

8. The method of any of claims 1 to 7, which is computer implemented.

9. The method of claim 8 wherein
a) input data is recorded on an electronic form on an local computer, wherein said input data comprises
i. time of intoxication and/or time of admission at the hospital
ii. values of
b) said input data is sent to a distant server
c) the end value is calculated on the distant server and
d) the information relating to the risk of adverse effect is sent back to the local computer [optionally with the end value].

10. The method of claim 9, wherein the communications between said the local computer and the distant server are encrypted.

11. Anti-colchicine antibodies or colchicine-binding fragments thereof for their use for the treatment of a patient intoxicated with colchicine, wherein the end value obtained for the patient by the method of any one of claim 1 to 10 indicates that the patient is susceptible to die.

12. The anti-colchicine antibodies for use according to claim 11, wherein the antibodies are polyclonal antibodies.

13. The anti-colchicine antibodies for use according to claim 11, wherein the antibodies are monoclonal antibodies.

14. The anti-colchicine antibodies for use according to any one of claims 11 to 13, wherein the antibodies are Fab fragments.

15. A method for obtaining a function to predict death of a patient intoxicated with colchicine, comprising the steps of:
i) classification of a cohort of patients in different groups according to the occurrence of death of the patients, and further taking into account the time of intoxication and/or hospital admission
ii) identification and selection of factors (amount of biochemical markers) which differ significantly between these groups by unidimensional analysis, wherein at least white blood cell count (GB), platelet count (PI) and at least one of plasma blood urea nitrogen (UREA) and blood bicarbonate ions (alkaline reserve, RA) are selected in this step
iii) logistic regression or Cox regression analysis assessings the independent discriminative value of markers for occurrence of death of the patient
iv) construction of the function by combination of these identified independent factors to thereby obtaining a Cox or logistic function to predict death of a patient intoxicated with colchicine.
